# EUROPÄISCHE PATENTSCHRIFT

(11) **EP 1 904 033 B1**
(45) Veröffentlichungstag und Bekanntmachung des Hinweises auf die Patenterteilung: **09.09.2009**
(21) Anmeldenummer: 06762653.1
(22) Anmeldetag: 17.07.2006
(51) Int. Cl.: A61K 9/16, A61K 31/64

(54) **VERFAHREN ZUR HERSTELLUNG VON GLIMEPIRID ENTHALTENDEN PHARMAZEUTISCHEN ZUSAMMENSETZUNGEN**
METHOD FOR PRODUCING PHARMACEUTICAL COMPOSITIONS CONTAINING GLIMEPIRIDE
PROCEDE DE PRODUCTION DE COMPOSITIONS PHARMACEUTIQUES CONTENANT DU GLIMEPIRIDE

(30) Priorität: 20.07.2005 DE 102005034484
(43) Veröffentlichungstag der Anmeldung: 02.04.2008
(73) Patentinhaber: Alfred E. Tiefenbacher GmbH & Co. KG, 22767 Hamburg (DE); USV Limited, Govandi, Mumbai 400 088 MAH (IN)
(72) Erfinder: SINGNURKAR, Prurushottam, S., Navi Mumbai 400 706 (IN); TEWARI, Prashant, Kumar, Bandra Mumbai 400 050 (IN); KORITKE, Claudia, 22607 Hamburg (DE); STARK, Georg, 25436 Uetersen (DE)
(74) Vertreter: Wittkopp, Alexander
(86) Internationale Anmeldenummer: PCT/EP2006/007016
(87) Internationale Veröffentlichungsnummer: WO 2007/009726

(56) Entgegenhaltungen:
- DE-A1- 19 860 698
- US-A1- 2003 147 952
- US-A1- 2004 147 564

## Beschreibung

Die Erfindung betrifft ein Verfahren zur Herstellung von Glimepirid enthaltenden festen, oralen pharmazeutischen Zusammensetzungen.

Glimepirid, dessen chemische Bezeichnung 1-({4-[2-(3-Ethyl-4-methyl-2-oxo-3-pyrrolin-1-carboxamido)-ethyl]phenyl}sulfonyl-3-(4-methylcyclohexyl)harnstoff lautet, ist ein orales Antidiabetikum aus der Gruppe der Sulfonylharnstoffe. Sulfonylharnstoffe bewirken eine Freisetzung vorhandenen Insulins aus den B-Zellen des Pankreas, verbessern gleichzeitig die Ansprechbarkeit auf den physiologischen Glucosereiz und wirken so antidiabetisch. Glimepirid sowie diesen Wirkstoff enthaltende pharmazeutische Zusammensetzungen wurden erstmals in DE 29 51 135 beschrieben. Auch US 2004/0147564 beschreibt tablettierte Glimepirid Zusammensetzungen. Als bevorzugte pharmazeutische Zusammensetzungen werden in DE 29 51 135 neben Glimepirid übliche Träger- und Hilfsstoffe (wie etwa Talkum, Stärke, Milchzucker oder Magnesiumstearat) enthaltende Tabletten gelehrt. Das zurzeit kommerziell unter der Kennzeichnung Amaryl^{®} (Aventis) vertriebene Glimepirid ist in Form von Tabletten erhältlich, die zur Behandlung des nicht-insulinabhängigen Diabetes mellitus (NIDDM; Typ-II-Diabetes) eingesetzt werden. Nachteilig an diesen Tabletten ist, dass sie die heute geltenden Anforderungen an die Stabilität, die für die Beantragung einer Arzneimittelzulassung erfüllt werden müssen, gerade noch erfüllen. Unter Stabilität versteht man nach der international anerkannten APV (Arbeitsgemeinschaft für pharmazeutische Verfahrenstechnik)-Richtlinie "Die spezifikationsgerechte Qualität des Arzneimittels bis zum Ende der vom Hersteller festgelegten Laufzeit". Die Qualität des Arzneimittels wird dabei durch den Wirkstoffgehalt und die Reinheit, die sensorisch wahrnehmbaren, physikalisch-chemischen und die mikrobiologischen Eigenschaften bestimmt. Der Wirkstoffgehalt soll bis zum Ende der Laufzeit 90 % des deklarierten Wertes nicht unterschreiten, d. h., Abbauprodukte bzw. Verunreinigungen, die eine Gefährdung der Patienten darstellen könnten, sollen ein Höchstmaß nicht überschreiten. Die Anforderungen, wie die entsprechende Stabilitätsprüfung zu erfolgen hat und wie hoch der Anteil an Abbauprodukten bzw. Verunreinigungen in dem zuzulassenden Arzneimittel sein darf, sind beispielsweise in den von der europäischen Zulassungsbehörde (EMEA) herausgegebenen Richtlinien (Guidelines) zur Stabilitätsprüfung (CPMP/QWP/122/02 vom 17. Dezember 2003) und zu Verunreinigungen in neuen Arzneimitteln (CPMP/ ICH/2738/99 vom 20. Februar 2003) festgelegt. Nach der letztgenannten Richtlinie ist beim Glimepirid eine Verunreinigung mit einem Anteil von 0,83 % zu qualifizieren, d. h., es müssen umfangreiche toxikologische Untersuchungen hinsichtlich dieser Verunreinigung vorgenommen werden. Auch erweisen sich Gesamt-Verunreinigungen von mehr als 1 % hinsichtlich des arzneimittelrechtlichen Zulassungsverfahrens als äußerst problematisch.

Es wäre daher wünschenswert, über Glimepirid enthaltende feste, orale pharmazeutische Zusammensetzungen mit verbesserter Stabilität zu verfügen.

Aufgabe der vorliegenden Erfindung ist es damit, Glimepirid enthaltende feste, orale pharmazeutische Zusammensetzungen mit verbesserter Stabilität bereitzustellen.

In den der Erfindung zugrundeliegenden Experimenten wurde überraschend gefunden, dass Glimepirid enthaltende feste, orale pharmazeutische Zusammensetzungen zu stabileren Zusammensetzungen führen, wenn diese nach einem Verfahren hergestellt werden, das folgende Schritte umfasst:
a) Herstellen einer Vormischung aus dem Wirkstoff und einem Hilfsstoff, der ein Bindemittel und/oder ein Netzmittel ist,
b) Herstellen einer Mischung umfassend die Vormischung und mindestens einen Hilfsstoff und gegebenenfalls
c) Verarbeiten der in Schritt b) hergestellten Mischung zur endgültigen Darreichungsform.

Dabei zeigen die nach dem erfindungsgemäßen Verfahren hergestellten Glimepirid enthaltenden festen, oralen pharmazeutischen Zusammensetzungen sowohl gegenüber dem Handelsprodukt "Amaryl^{®}" als auch gegenüber solchen Zusammensetzungen, die in herkömmlicher Weise durch einfaches Mischen geeigneter Hilfsstoffe mit dem wirksamen Bestandteil, also ohne Herstellung der zuvor genannten Vormischung und deren Weiterverarbeitung hergestellt werden, eine verbesserte Stabilität.

Das erfindungsgemäße Verfahren wird vorzugsweise so durchgeführt, dass zur Herstellung der Vormischung aus Glimepirid und dem pharmazeutischen Hilfsstoff Povidon (Polyvinylpyrrolidon, PVP) verwendet wird. Gemäß einer besonders bevorzugten Ausführungsform werden sowohl das Glimepirid als auch das Povidon in mikronisierter Form zur Herstellung der Vormischung verwendet.

Das erfindungsgemäße Verfahren ist jedoch nicht auf die Verwendung spezieller Binde- und/oder Netzmittel zur Herstellung der Vormischung beschränkt, vielmehr können alle üblicherweise verwendeten Binde- und Netzmittel verwendet werden. Hierbei sind beispielsweise Stärke, Gelatine, Saccharose, Glucose, Sorbitol, mikrokristalline Cellulose, Methylcellulose, Ethylcellulose, Hydroxypropylcellulose, Hydroxypropylmethylcellulose und Polyethylenglykol als geeignete Bindemittel sowie Carbonsäureester und -ether, Alkyl-, Alkylaryl- und Fettalkoholsulfate und -sulfonate, Alkylphenolethoxylate, Betaine, (ethoxylierte) Fettalkohole, Sorbitan- und Zuckerester als geeignete Netzmittel zu nennen. Vorzugsweise wird ein Hilfsstoff verwendet, der sowohl als Bindemittel als auch als Netzmittel fungiert. Diesbezüglich sind beispielsweise Methylcellulose, Carboxymethylcellulose, Carboxymethylstärke und Polyethylenglykol zu nennen. Vorzugsweise wird zur Herstellung der Vormischung mikronisierter Hilfsstoff verwendet.

Erfindungsgemäß kann das verwendete Glimepirid als solches oder in Form seiner Salze eingesetzt werden. Vorzugsweise wird der Wirkstoff in mikronisierter Form verwendet.

Gemäß einer weiteren bevorzugten Ausführungsform wird die Vormischung mit Lactose als Hilfsstoff gemischt, um die zuvor unter Schritt b) des erfindungsgemäßen Verfahrens genannte Mischung umfassend die Vormischung und mindestens einen Hilfsstoff herzustellen. Gemäß einer besonders bevorzugten Ausführungsform wird hierbei ein Lactose-Granulat eingesetzt. Jedoch ist das erfindungsgemäße Verfahren nicht auf die Verwendung spezieller Hilfsstoffe zur Herstellung der Mischung umfassend die Vormischung und mindestens einen Hilfsstoff beschränkt. Diesbezüglich können alle üblichen pharmazeutischen Hilfsstoffe wie Füllmittel (z. B. Lactose, Mannitol, Dextrose, Dicalciumphosphat), Bindemittel (z. B. Stärke, Gelatine, Saccharose, Glucose, Sorbitol, mikrokristalline Cellulose, Methylcellulose, Ethylcellulose, Hydroxypropylcellulose, Hydroxypropylmethylcellulose, Polyethylenglykol), Sprengmittel (z. B. Natriumstärkeglykolat, Natriumcarboxymethylcellulose, Stärke, modifizierte Maisstärke, quervernetztes Polyvinylpyrrolidon), Schmiermittel (z. B. Magnesiumstearat, Talkum, Natriumlaurylsulfat) und dergleichen zum Einsatz kommen.

Erfindungsgemäß wird die Mischung umfassend die Vormischung und den/die Hilfsstoff/e hergestellt, indem diese Komponenten in einem geeigneten Mischer gemischt werden.

Diese Mischung kann jedoch auch hergestellt werden, indem die Vormischung zu einer Granuliermischung gegeben wird.

Die nach dem erfindungsgemäßen Verfahren gemäß Schritt b) hergestellte Mischung umfassend die Vormischung und den/die Hilfsstoff/e liegt vorzugsweise in Form eines Pulvers, Granulats oder von Pellets vor.

Die gemäß Schritt b) des erfindungsgemäßen Verfahrens hergestellte Mischung umfassend die Vormischung und den/die Hilfsstoff/e kann gegebenenfalls nach üblichen Verfahren zur endgültigen Darreichungsform verarbeitet werden. Als endgültige Darreichungsformen kommen vorzugsweise Tabletten, Kapseln, Sachets und Dragees in Betracht. Besonders bevorzugt wird die Mischung zu Tabletten als endgültige Darreichungsform verpresst.

Die nachfolgenden Beispiele veranschaulichen die Erfindung.

### Referenzbeispiel

### Herstellung von Glimepirid enthaltenden Tabletten ohne Vormischung des Wirkstoffs

Aus 7 kg Povidon, 1,34 kg Polysorbat 80, 4,66 kg Maisstärke und 32,5 kg Wasser wird eine homogene Granulierungspaste hergestellt. 149,50 kg Lactose-Monohydrat, 6,66 kg Natriumcarboxymethylstärke und 0,3 kg Eisenoxid werden gesiebt und in einem Mixer entsprechender Größe gemischt. Die so hergestellte Mischung wird mit der zuvor hergestellten Granulierungspaste so lange vermischt, bis die Masse homogen ist, und anschließend getrocknet, wobei ein Lactose-Granulat erhalten wird. Schrittweise werden das Lactose-Granulat, 2 kg Talkum, 1,2 kg Magnesiumstearat, 3,34 kg Natriumcarboxymethylstärke und 3 kg mikronisiertes Glimepirid vermischt. Die dabei erhaltene Mischung wird auf einer handelsüblichen Rundläufer-Tablettiermaschine zu Tabletten verpresst, die 1, 2 oder 3 mg Glimepirid als wirksamen Bestandteil enthalten.

### Beispiel 1

3 kg mikronisiertes Povidon und 3 kg mikronisiertes Glimepirid werden in einem geeigneten Mixer homogenisiert, wobei eine Vormischung erhalten wird. Aus 7 kg Povidon, 1,34 kg Polysorbat 80, 4,66 kg Maisstärke und 32,5 kg Wasser wird eine homogene Granulierungspaste hergestellt. 149,50 kg Lactose-Monohydrat, 6,66 kg Natriumcarboxymethylstärke und 0,3 kg Eisenoxid werden gesiebt und in einem Mixer entsprechender Größe gemischt. Die dabei erhaltene Mischung wird mit der Granulierungspaste so lange vermischt, bis die Masse homogen ist, und anschließend getrocknet, wobei ein Lactose-Granulat erhalten wird. Schrittweise werden das Lactose-Granulat, 2 kg Talkum, 1,2 kg Magnesiumstearat, 3,34 kg Natriumcarboxymethylstärke und die Povidon/Glimepirid-Vormischung vermischt. Die dabei erhaltene Mischung wird auf einer handelsüblichen Rundläufer-Tablettiermaschine zu Tabletten verpresst, die 1, 2 oder 3 mg Glimepirid als wirksame Bestandteile enthalten.

### Beispiel 2

Die gemäß dem Beispiel 1 hergestellten Tabletten wurden unter normalen Lagerungsbedingungen bei 25 ± 2 °C und 60 ± 5 % relativer Luftfeuchtigkeit 1, 3, 6 oder 12 Monate gelagert. Die Tabletten zeigten nach der angegebenen Lagerzeit keinerlei Verfärbung. Mittels HPLC-Untersuchungen wurde auf Verunreinigungen in den Tabletten vor und nach der Lagerung untersucht. Zum Vergleich wurden identische Untersuchungen mit den gemäß dem Referenzbeispiel hergestellten Tabletten und den im Handel befindlichen Tabletten durchgeführt. Untersuchungen hatten gezeigt, dass die im Handel befindlichen Tabletten in üblicherweise durch einfaches Mischen des Wirkstoffes mit den Hilfsstoffen hergestellt werden. Die erhaltenen Ergebnisse aus den Stabilitätsuntersuchungen sind in den nachfolgenden Tabellen 1-12 zusammengefasst.

### Tabellen 1-12:

Verunreinigungen in den erfindungsgemäß hergestellten Tabletten (Beispiel 1), den herkömmlich hergestellten Tabletten (Referenzbeispiel) und den im Handel befindlichen Tabletten nach Lagerung unter den angegebenen Bedingungen. Die Abkürzung "rrt" in den Tabellen bedeutet die relative Retentionszeit.

### Tabellen 1-4:

1 mg Glimepirid enthaltende Tabletten wurden bei 25 ± 2 °C und 60 ± 5 % relativer Luftfeuchtigkeit 1 Monat, 3 Monate, 6 Monate und 12 Monate gelagert.

**Tabelle 1: Lagerzeit: 1 Monat**

| **Tabletten** | **Spezifikation** |
|---|---|
| gemäß Beispiel 1 | *Bekannte Verunreinigungen:* Sulfonamid (rrt: 0,20): 0,06 % Carbamat (rrt: 0,28): 0,01 % *Unbekannte Verunreinigungen, einzeln (rrt: 0,66):* 0,03 % *Unbekannte Verunreinigungen, gesamt:* 0,05 % *Gesamte Verunreinigungen:* 0,12 % |
| gemäß Referenzbeispiel | *Bekannte Verunreinigungen:* Sulfonamid (rrt: 0,20): 0,31 % Carbamat (rrt: 0,28): 0,05 % *Unbekannte Verunreinigungen, einzeln (rrt: 0,66):* 0,017 % *Unbekannte Verunreinigungen, gesamt:* 0,21 % *Gesamte Verunreinigungen:* 0,57 % |
| im Handel befindliche | *Bekannte Verunreinigungen:* Sulfonamid (rrt: 0,20): 0,36 % Carbamat (rrt: 0,28): 0,08 % *Unbekannte Verunreinigungen, einzeln (rrt: 0,66):* 0,02 % *Unbekannte Verunreinigungen, gesamt:* 0,15 % *Gesamte Verunreinigungen:* 0,59 % |

**Tabelle 2: Lagerzeit: 3 Monate**

| **Tabletten** | **Spezifikation** |
|---|---|
| gemäß Beispiel 1 | *Bekannte Verunreinigungen:* Sulfonamid (rrt: 0,20): 0,08 % Carbamat (rrt: 0,28): 0,03 % *Unbekannte Verunreinigungen, einzeln (rrt: 0, 66):* 0,03 % *Unbekannte Verunreinigungen, gesamt:* 0,11 % *Gesamte Verunreinigungen:* 0,22 % |
| gemäß Referenzbeispiel | *Bekannte Verunreinigungen:* Sulfonamid (rrt: 0,20): 0,32 % Carbamat (rrt: 0,28): 0,04 % *Unbekannte Verunreinigungen, einzeln (rrt: 0,66):* 0,03 % *Unbekannte Verunreinigungen, gesamt:* 0,14 % *Gesamte Verunreinigungen:* 0,50 % |
| im Handel befindliche | *Bekannte Verunreinigungen:* Sulfonamid (rrt: 0,20): 0,37 % Carbamat (rrt: 0,28): 0,06 % *Unbekannte Verunreinigungen, einzeln (rrt: 0,66):* 0,02 % *Unbekannte Verunreinigungen, gesamt:* 0,17 % *Gesamte Verunreinigungen:* 0,60 % |

**Tabelle 3: Lagerzeit: 6 Monate**

| **Tabletten** | **Spezifikation** |
|---|---|
| gemäß Beispiel 1 | *Bekannte Verunreinigungen:* Sulfonamid (rrt: 0,20): 0,12 % Carbamat (rrt: 0,28): 0,02 % *Unbekannte Verunreinigungen, einzeln (rrt: 0, 66):* 0,01 % *Unbekannte Verunreinigungen, gesamt:* 0,11 % *Gesamte Verunreinigungen:* 0,25 % |
| gemäß Referenzbeispiel | *Bekannte Verunreinigungen:* Sulfonamid (rrt: 0,20): 0,38 % Carbamat (rrt: 0,28): 0,03 % *Unbekannte Verunreinigungen, einzeln (rrt: 0,66):* 0,02 % *Unbekannte Verunreinigungen, gesamt:* 0,14 % *Gesamte Verunreinigungen:* 0,55 % |
| im Handel befindliche | *Bekannte Verunreinigungen:* Sulfonamid (rrt: 0,20): 0,48 % Carbamat (rrt: 0,28): 0,05 % *Unbekannte Verunreinigungen, einzeln (rrt: 0,66):* 0,02 % *Unbekannte Verunreinigungen, gesamt:* 0,15 % *Gesamte Verunreinigungen:* 0,68 % |

**Tabelle 4: Lagerzeit: 12 Monate**

| **Tabletten** | **Spezifikation** |
|---|---|
| gemäß Beispiel 1 | *Bekannte Verunreinigungen:* Sulfonamid (rrt: 0,20): 0,13 % Carbamat (rrt: 0,28): 0,01 % *Unbekannte Verunreinigungen, einzeln (rrt: 0, 66):* 0,04 % *Unbekannte Verunreinigungen, gesamt:* 0,08 % *Gesamte Verunreinigungen:* 0,22 % |
| gemäß Referenzbeispiel | *Bekannte Verunreinigungen:* Sulfonamid (rrt: 0,20): 0,41 % Carbamat (rrt: 0,28):0,03 % *Unbekannte Verunreinigungen, einzeln (rrt: 0,66):* 0,04 % *Unbekannte Verunreinigungen, gesamt:* 0,17 % *Gesamte Verunreinigungen:* 0,61 % |
| im Handel befindliche | *Bekannte Verunreinigungen:* Sulfonamid (rrt: 0,20): 0,51 % Carbamat (rrt: 0,28): 0,05 % *Unbekannte Verunreinigungen, einzeln (rrt: 0, 66):* 0,03 % *Unbekannte Verunreinigungen, gesamt: 0,16* % *Gesamte Verunreinigungen:* 0,72 % |

### Tabellen 5-8:

2 mg Glimepirid enthaltende Tabletten wurden bei 25 ± 2 °C und 60 ± 5 % relativer Luftfeuchtigkeit 1 Monat, 3 Monate, 6 Monate und 12 Monate gelagert.

**Tabelle 5: Lagerzeit: 1 Monat**

| **Tabletten** | **Spezifikation** |
|---|---|
| gemäß Beispiel 1 | *Bekannte Verunreinigungen:* Sulfonamid (rrt: 0,20): 0,05 % Carbamat (rrt: 0,28): 0,01 % *Unbekannte Verunreinigungen, einzeln (rrt: 0, 66):* 0,02 % *Unbekannte Verunreinigungen, gesamt:* 0,05 % *Gesamte Verunreinigungen:* 0,11 % |
| gemäß Referenzbeispiel | *Bekannte Verunreinigungen:* Sulfonamid (rrt: 0,20): 0,31 % Carbamat (rrt: 0,28): 0,05 % *Unbekannte Verunreinigungen, einzeln (rrt: 0,66):* 0,02 % *Unbekannte Verunreinigungen, gesamt:* 0,13 % *Gesamte Verunreinigungen:* 0,49 % |
| im Handel befindliche | *Bekannte Verunreinigungen:* Sulfonamid (rrt: 0,20): 0,32 % Carbamat (rrt: 0,28): 0,11 % *Unbekannte Verunreinigungen, einzeln (rrt: 0,66):* 0,02 % *Unbekannte Verunreinigungen, gesamt:* 0,13 % *Gesamte Verunreinigungen:* 0,56 % |

**Tabelle 6: Lagerzeit: 3 Monate**

| **Tabletten** | **Spezifikation** |
|---|---|
| gemäß Beispiel 1 | *Bekannte Verunreinigungen:* Sulfonamid (rrt: 0,20): 0,09 % Carbamat (rrt: 0,28): 0,01 % *Unbekannte Verunreinigungen, einzeln (rrt: 0,66):* 0,03 % *Unbekannte Verunreinigungen, gesamt:* 0,09 % *Gesamte Verunreinigungen:* 0,19 % |
| gemäß Referenzbeispiel | *Bekannte Verunreinigungen:* Sulfonamid (rrt: 0,20): 0,36 % Carbamat (rrt: 0,28): 0,09 % *Unbekannte Verunreinigungen, einzeln (rrt: 0, 66):* 0,03 % *Unbekannte Verunreinigungen, gesamt:* 0,17 % *Gesamte Verunreinigungen:* 0,62 % |
| im Handel befindliche | *Bekannte Verunreinigungen:* Sulfonamid (rrt: 0,20): 0,44 % Carbamat (rrt: 0,28): 0,11 % *Unbekannte Verunreinigungen, einzeln (rrt: 0, 66):* 0,03 % *Unbekannte Verunreinigungen, gesamt:* 0,18 % *Gesamte Verunreinigungen:* 0,73 % |

**Tabelle 7: Lagerzeit: 6 Monate**

| **Tabletten** | **Spezifikation** |
|---|---|
| gemäß Beispiel 1 | *Bekannte Verunreinigungen:* Sulfonamid (rrt: 0,20): 0,10 % Carbamat (rrt: 0,28): 0,01 % *Unbekannte Verunreinigungen, einzeln (rrt: 0,66):* 0,01 % *Unbekannte Verunreinigungen, gesamt:* 0,10 % *Gesamte Verunreinigungen:* 0,21 % |
| gemäß Referenzbeispiel | *Bekannte Verunreinigungen:* Sulfonamid (rrt: 0,20): 0,45 % Carbamat (rrt: 0,28): 0,06 % *Unbekannte Verunreinigungen, einzeln (rrt: 0,66):* 0,02 % *Unbekannte Verunreinigungen, gesamt:* 0,12 % *Gesamte Verunreinigungen:* 0,63 % |
| im Handel befindliche | *Bekannte Verunreinigungen:* Sulfonamid (rrt: 0,20): 0,54 % Carbamat (rrt: 0,28): 0,06 % *Unbekannte Verunreinigungen, einzeln (rrt: 0,66):* 0,03 % *Unbekannte Verunreinigungen, gesamt:* 0,15 % *Gesamte Verunreinigungen:* 0,75 % |

**Tabelle 8: Lagerzeit: 12 Monate**

| **Tabletten** | **Spezifikation** |
|---|---|
| gemäß Beispiel 1 | *Bekannte Verunreinigungen:* Sulfonamid (rrt: 0,20): 0,12 % Carbamat (rrt: 0,28): 0,01 % *Unbekannte Verunreinigungen, einzeln (rrt: 0, 66):* 0,05 % *Unbekannte Verunreinigungen, gesamt:* 0,07 % *Gesamte Verunreinigungen:* 0,20 % |
| gemäß Referenzbeispiel | *Bekannte Verunreinigungen:* Sulfonamid (rrt: 0,20): 0,53 % Carbamat (rrt: 0,28): 0,06 % *Unbekannte Verunreinigungen, einzeln (rrt: 0, 66):* 0,03 % *Unbekannte Verunreinigungen, gesamt:* 0,15 % *Gesamte Verunreinigungen:* 0,74 % |
| im Handel befindliche | *Bekannte Verunreinigungen:* Sulfonamid (rrt: 0,20): 0,60 % Carbamat (rrt: 0,28): 0,06 % *Unbekannte Verunreinigungen, einzeln (rrt: 0,66):* 0,04 % *Unbekannte Verunreinigungen, gesamt:* 0,17 % *Gesamte Verunreinigungen:* 0,83 % |

### Tabellen 9-12:

3 mg Glimepirid enthaltende Tabletten wurden bei 25 ± 2 °C und 60 ± 5 % relativer Luftfeuchtigkeit 1 Monat, 3 Monate, 6 Monate und 12 Monate gelagert.

**Tabelle 9: Lagerzeit: 1 Monat**

| **Tabletten** | **Spezifikation** |
|---|---|
| gemäß Beispiel 1 | *Bekannte Verunreinigungen:* Sulfonamid (rrt: 0,20): 0,05 % Carbamat (rrt: 0,28):0,01 % *Unbekannte Verunreinigungen, einzeln (rrt: 0, 66):* 0,01 % *Unbekannte Verunreinigungen, gesamt:* 0,04 % *Gesamte Verunreinigungen:* 0,10 % |
| gemäß Referenzbeispiel | *Bekannte Verunreinigungen:* Sulfonamid (rrt: 0,20): 0,27 % Carbamat (rrt: 0,28): 0,06 % *Unbekannte Verunreinigungen, einzeln (rrt: 0,66):* 0,03 % *Unbekannte Verunreinigungen, gesamt:* 0,17 % *Gesamte Verunreinigungen:* 0,50 % |
| im Handel befindliche | *Bekannte Verunreinigungen:* Sulfonamid (rrt: 0,20): 0,26 % Carbamat (rrt: 0,28): 0,08 % *Unbekannte Verunreinigungen, einzeln (rrt: 0,66):* 0,08 % *Unbekannte Verunreinigungen, gesamt:* 0,18 % *Gesamte Verunreinigungen:* 0,52 % |

**Tabelle 10: Lagerzeit: 3 Monate**

| **Tabletten** | **Spezifikation** |
|---|---|
| gemäß Beispiel 1 | *Bekannte Verunreinigungen:* Sulfonamid (rrt: 0,20): 0,07 % Carbamat (rrt: 0,28): 0,02 % *Unbekannte Verunreinigungen, einzeln (rrt: 0,66):* 0,03 % *Unbekannte Verunreinigungen, gesamt:* 0,08 % *Gesamte Verunreinigungen:* 0,17 % |
| gemäß Referenzbeispiel | *Bekannte Verunreinigungen:* Sulfonamid (rrt: 0,20): 0,30 % Carbamat (rrt: 0,28): 0,06 % *Unbekannte Verunreinigungen, einzeln (rrt: 0,66):* 0,04 % *Unbekannte Verunreinigungen, gesamt:* 0,18 % *Gesamte Verunreinigungen:* 0,54 % |
| im Handel befindliche | *Bekannte Verunreinigungen:* Sulfonamid (rrt: 0,20): 0,31 % Carbamat (rrt: 0,28): 0,08% *Unbekannte Verunreinigungen, einzeln (rrt: 0,66):* 0,02 % *Unbekannte Verunreinigungen, gesamt:* 0,18 % *Gesamte Verunreinigungen:* 0,57 % |

**Tabelle 11: Lagerzeit: 6 Monate**

| **Tabletten** | **Spezifikation** |
|---|---|
| gemäß Beispiel 1 | *Bekannte Verunreinigungen:* Sulfonamid (rrt: 0,20): 0,09 % Carbamat (rrt: 0,28): 0,01 % *Unbekannte Verunreinigungen, einzeln (rrt: 0, 66):* 0,01 % *Unbekannte Verunreinigungen, gesamt:* 0,09 % *Gesamte Verunreinigungen:* 0,19 % |
| gemäß Referenzbeispiel | *Bekannte Verunreinigungen:* Sulfonamid (rrt: 0,20): 0,34 % Carbamat (rrt: 0,28): 0,04 % *Unbekannte Verunreinigungen, einzeln (rrt: 0, 66):* 0,01 % *Unbekannte Verunreinigungen, gesamt:* 0,18 % *Gesamte Verunreinigungen:* 0,56 % |
| im Handel befindlichen | *Bekannte Verunreinigungen:* Sulfonamid (rrt: 0,20): 0,35 % Carbamat (rrt: 0,28): 0,06 % *Unbekannte Verunreinigungen, einzeln (rrt: 0,66):* 0,02 % *Unbekannte Verunreinigungen, gesamt:* 0,15 % *Gesamte Verunreinigungen:* 0,56 % |

**Tabelle 12: Lagerzeit: 12 Monate**

| **Tabletten** | **Spezifikation** |
|---|---|
| gemäß Beispiel 1 | *Bekannte Verunreinigungen:* Sulfonamid (rrt: 0,20): 0,12 % Carbamat (rrt: 0,28): 0,01 *Unbekannte Verunreinigungen, einzeln (rrt: 0,66):* 0,04 % *Unbekannte Verunreinigungen, gesamt:* 0,06 % *Gesamte Verunreinigungen:* 0,19 % |
| gemäß Referenzbeispiel | *Bekannte Verunreinigungen:* Sulfonamid (rrt: 0,20): 0,41 % Carbamat (rrt: 0,28): 0,04 % *Unbekannte Verunreinigungen, einzeln (rrt: 0,66):* 0,04 % *Unbekannte Verunreinigungen, gesamt:* 0,15 % *Gesamte Verunreinigungen:* 0,60 % |
| im Handel befindliche | *Bekannte Verunreinigungen:* Sulfonamid (rrt: 0,20): 0,41 % Carbamat (rrt: 0,28): 0,06 % *Unbekannte Verunreinigungen, einzeln (rrt: 0,66):* 0,02 % *Unbekannte Verunreinigungen, gesamt:* 0,13 % *Gesamte Verunreinigungen:* 0,60 % |

Wie aus den Tabellen ersichtlich wird, sind die Sulfonamid-Verunreinigung sowie die Gesamt-Verunreinigungen bei den erfindungsgemäß hergestellten Tabletten deutlich niedriger als bei den nach dem herkömmlichen Verfahren hergestellten Tabletten sowie den im Handel befindlichen Tabletten. Beispielsweise entstehen nach 12 Monaten Lagerung bei den erfindungsgemäß hergestellten Tabletten 0,12-0,13 % der Sulfonamid-Verunreinigung sowie 0,19-0,22 % an Gesamt-Verunreinigungen. Bei den nach dem herkömmlichen Verfahren hergestellten Tabletten sowie den im Handel befindlichen Tabletten entstehen dagegen nach 12 Monaten Lagerung 0,41-0,53 % (Referenzbeispiel) sowie 0,41-0,60 % (im Handel befindliche Tabletten) der Sulfonamid-Verunreinigung und 0,60-0,74 % (Referenzbeispiel) sowie 0,60-0,83 % (im Handel befindliche Tabletten) an Gesamt-Verunreinigungen.

Die in den Tabellen zusammengefassten Daten der Stabilitätsuntersuchungen zeigen damit eindrucksvoll, dass durch das erfindungsgemäße Verfahren Glimepirid enthaltende feste, orale pharmazeutische Zusammensetzungen mit verbesserter Stabilität zur Verfügung gestellt werden.

## Patentansprüche

1. Verfahren zur Herstellung von Glimepirid enthaltenden festen, oralen pharmazeutischen Zusammensetzungen, wobei das Verfahren folgende Schritte umfasst:
a) Herstellen einer Vormischung aus dem mikronisierten Wirkstoff und einem Hilfsstoff, der ein Bindemittel und/oder ein Netzmittel ist,
b) Herstellen einer Mischung umfassend die Vormischung und mindestens einen Hilfsstoff, der ein Lactose-Granulat ist, das Natriumcarboxymethylstärke, Polysorbat, Povidon und Maisstärke umfasst und gegebenenfalls
c) Verarbeiten der in Schritt b) hergestellten Mischung zur endgültigen Darreichungsform.

2. Verfahren nach Anspruch 1 wobei der in Schritt a) verwendete Hilfsstoff in mikronisierter Form eingesetzt wird.

3. Verfahren nach einem der Ansprüche 1-2, wobei der in Schritt a) verwendete Hilfsstoff Povidon ist.

4. Verfahren nach einem der Ansprüche 1-3, wobei die in Schritt b) hergestellte Mischung in Form eines Pulvers vorliegt.

5. Verfahren nach einem der Ansprüche 1-3, wobei die in Schritt b) hergestellte Mischung in Form von Granulaten vorliegt.

6. Verfahren nach einem der Ansprüche 1-3, wobei die in Schritt b) hergestellte Mischung in Form von Pellets vorliegt.

7. Verfahren nach einem der Ansprüche 1-6, wobei die in Schritt b) hergestellte Mischung zu Tabletten als endgültige Darreichungsform verpresst wird.

8. Verfahren nach einem der Ansprüche 1-6, wobei die in Schritt b) hergestellte Mischung in eine Kapsel als endgültige Darreichungsform abgefüllt wird.

## Claims

1. Method for the preparation of solid, oral pharmaceutical compositions comprising glimepiride, whereby the method comprises the following steps:
a) preparation of a pre-mixture of the micronized active ingredient and an excipient, which is a binder and/or a wetting agent,
b) preparation of a mixture comprising the pre-mixture and at least one excipient, which is a lactose granulate comprising sodium carboxymethyl starch, polysorbate, povidon and maize starch, and optionally
c) converting the mixture obtained in step b) into the final dosage form.

2. Method according to claim 1, whereby the excipient used in step a) is used in micronized form.

3. Method according to claim 1 or claim 2, whereby the excipient used in step a) is povidone.

4. Method according to anyone of claims 1-3, whereby the mixture prepared in step b) is in form of a powder.

5. Method according to anyone of claims 1-3, whereby the mixture prepared in step b) is in form of granulates.

6. Method according to anyone of claims 1-3, whereby the mixture prepared in step b) is in form of pellets.

7. Method according to anyone of claims 1-6, whereby the mixture prepared in step b) is compressed to tablets as final dosage form.

8. Method according to anyone of claims 1-6, whereby the mixture prepared in step b) is filled into capsules as final dosage form.

## Revendications

1. Procédé de production de compositions pharmaceutiques solides administrables par voie orale contenant du glimépiride, ledit procédé comprenant les étapes suivantes :
a) production d'un prémélange composé de l'agent actif micronisé et d'un excipient, qui consiste en un liant et/ou un agent mouillant,
b) production d'un mélange comprenant le prémélange et au moins un excipient, qui consiste en un granulat à base de lactose, qui comprend du sodium carboxyméthylamidon, du polysorbate, de la povidone et de l'amidon de maïs, et éventuellement
c) transformation du mélange obtenu à l'étape b) en la forme galénique définitive.

2. Procédé selon la revendication 1, dans lequel l'excipient employé à l'étape a) est mis en oeuvre sous forme micronisée.

3. Procédé selon la revendication 1 ou la revendication 2, dans lequel l'excipient employé à l'étape a) est la povidone.

4. Procédé selon l'une quelconque des revendications 1 à 3, dans lequel le mélange obtenu à l'étape b) se présente sous forme pulvérulente.

5. Procédé selon l'une quelconque des revendications 1 à 3, dans lequel le mélange obtenu à l'étape b) se présente sous la forme de granulés.

6. Procédé selon l'une quelconque des revendications 1 à 3, dans lequel le mélange obtenu à l'étape b) se présente sous la forme de granules.

7. Procédé selon l'une quelconque des revendications 1 à 6, dans lequel le mélange obtenu à l'étape b) est compressé sous la forme de comprimés en vue d'obtenir la forme galénique définitive.

8. Procédé selon l'une quelconque des revendications 1 à 6, dans lequel le mélange obtenu à l'étape b) est mis en gélule en vue d'obtenir la forme galénique définitive.
